# EUROPÄISCHE PATENTSCHRIFT

(11) **EP 1 556 486 B1**
(45) Veröffentlichungstag und Bekanntmachung des Hinweises auf die Patenterteilung: **13.01.2010**
(21) Anmeldenummer: 03779858.4
(22) Anmeldetag: 06.11.2003
(51) Int. Cl.: C12N 15/11, C12N 5/10, A61K 31/7088

(54) **WIRKSAME UND STABILE DNA-ENZYME**
EFFECTIVE AND STABLE DNA ENZYMES
ENZYMES A ADN ACTIVES ET STABLES

(30) Priorität: 06.11.2002 DE 10251682; 15.05.2003 DE 10322662
(43) Veröffentlichungstag der Anmeldung: 27.07.2005
(73) Patentinhaber: Grünenthal GmbH, 52078 Aachen (DE)
(72) Erfinder: SCHUBERT, Steffen, 14059 Berlin (DE); KURRECK, Jens, 12205 Berlin (DE); GRÜNWELLER, Arnold, 12165 Berlin (DE); ERDMANN, Volker, 14163 Berlin (DE)
(74) Vertreter: Hiebl, Inge Elisabeth
(86) Internationale Anmeldenummer: PCT/EP2003/012413
(87) Internationale Veröffentlichungsnummer: WO 2004/042046

(56) Entgegenhaltungen:
- WO-A-02/18407
- WO-A-02/44321
- SIOUD M ET AL: "Design of nuclease resistant protein kinase calpha DNA enzymes with potential therapeutic application" JOURNAL OF MOLECULAR BIOLOGY, LONDON, GB, Bd. 296, Nr. 3, 25. Februar 2000 (2000-02-25), Seiten 937-947, XP004461588 ISSN: 0022-2836
- WARASHINA M ET AL: "Extremely high and specific activity of DNA enzymes in cells with a Philadelphia chromosome." CHEMISTRY & BIOLOGY. ENGLAND APR 1999, Bd. 6, Nr. 4, April 1999 (1999-04), Seiten 237-250, XP002272987 ISSN: 1074-5521
- BEIGELMAN L ET AL: "CHEMICAL MODIFICATION OF HAMMERHEAD RIBOZYMES CATALYTIC ACTIVITY AND NUCLEASE RESISTANCE" JOURNAL OF BIOLOGICAL CHEMISTRY, AMERICAN SOCIETY OF BIOLOGICAL CHEMISTS, BALTIMORE, MD, US, Bd. 270, Nr. 43, 27. Oktober 1995 (1995-10-27), Seiten 25702-25708, XP000601905 ISSN: 0021-9258
- SCHUBERT STEFFEN ET AL: "RNA cleaving '10-23' DNAzymes with enhanced stability and activity." NUCLEIC ACIDS RESEARCH. ENGLAND 15 OCT 2003, Bd. 31, Nr. 20, 15. Oktober 2003 (2003-10-15), Seiten 5982-5992, XP002272988 ISSN: 1362-4962
- GRÜNWELLER ARNOLD ET AL: "Comparison of different antisense strategies in mammalian cells using locked nucleic acids, 2'-O-methyl RNA, phosphorothioates and small interfering RNA." NUCLEIC ACIDS RESEARCH. 15 JUN 2003, Bd. 31, Nr. 12, 15. Juni 2003 (2003-06-15), Seiten 3185-3193, XP002286510 ISSN: 1362-4962
- NOVINA C D ET AL: "siRNA directed inhibition of HIV-1 infection" NATURE MEDICINE, NATURE PUBLISHING, CO, US, Bd. 7, Nr. 8, Juli 2002 (2002-07), Seiten 681-686, XP002955298 ISSN: 1078-8956
- BRUMMELKAMP T R ET AL: "STABLE SUPPRESSION OF TUMORIGENICITY BY VIRUS-MEDIATED RNA INTERFERENCE" CANCER CELL, XX, US, Bd. 2, Nr. 3, September 2002 (2002-09), Seiten 243-247, XP009006464 ISSN: 1535-6108

## Beschreibung

Die vorliegenden Erfindung betrifft DNA-Enzyme vom Typ 10-23, die durch Modifizierung bestimmter Nukleotide in der Kemsequenz besonders stabil sind und darüber hinaus im wesentlichen die gleiche oder eine höhere Spalteffizienz gegenüber ihrem Substrat aufweisen, wie die entsprechenden nicht-modifizierten DNA-Enzyme. Weitere Gegenstände der vorliegenden Anmeldung betreffen Wirtszellen, welche die erfindungsgemäßen DNA-Enzyme enthalten. Darüber hinaus wird ein Arzneimittel bereitgestellt, weiches die erfindungsgemäßen DNA-Enzyme bzw. Wirtszellen enthalten. Die DNA-Enzyme und weiteren Gegenstände sind insbesondere gegen den Vanilloid-Rezeptor 1 (VR1) bzw. Picomaviren gerichtet. Die Wirtszellen eignen sich als Arzneimittel bzw. zur Herstellung von Arzneimitteln, insbesondere zur Behandlung von Schmerzen und anderen mit VR1 zusammenhängenden krankhaften Zuständen.

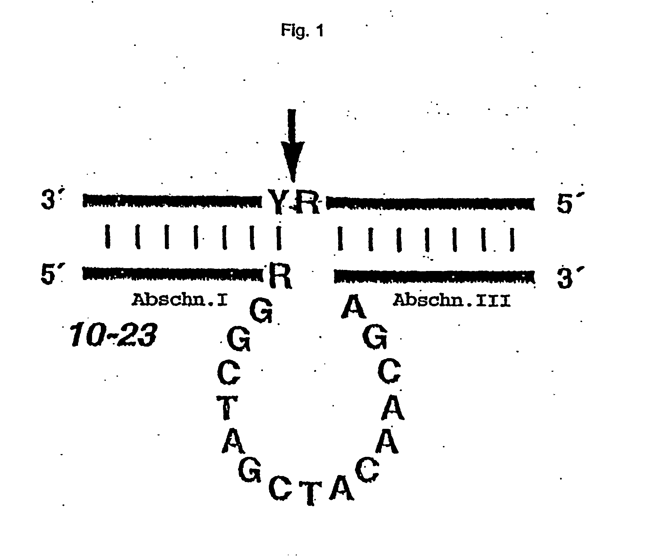

RNA-spattende DNA-Enzyme wurden ausgehend von Hammerhead-Ribozymen durch *in vitro* Selektionsexperimente entickelt. Im Vergleich zu RNA-Spezies sind DNA-Enzyme leichter herzustellen, und sie sind, insbesondere in biologischen Geweben, stabiler. Das im Stand der Technik bekannte DNA-Enzym mit der größten Spalteffizienz und der flexibelsten Substraterkennung ist das sogenannte DNA-Enzym vom Typ "10-23" (Santoro und Joice (1997) Proc. Natl. Acad. Sci. USA 94: 4262-4266). Das DNA-Enzym vom Typ 10-23 enthält eine katalytische Domäne (Kemsequenz) von 15 Nukleotiden, welche von zwei Substraterkennungsdomänen oder -armen, die jeweils 7-10 Nukleotide umfassen, flankiert wird (vgl. Fig. 1). Das DNA-Enzym vom Typ 10-23 bindet das RNA-Substrat durch Basenpaarung gemäß den Watson-Crick-Reglen über die Substraterkennungsarme.

Trotz der höheren Stabilität von DNA-Enzymen im Vergleich zu Ribozymen ist es insbesondere für *in vivo* Anwendungen, bspw. als Arzneimittel, erforderlich, diese Moleküle gegenüber nükleolytischen Angriffen zu stabilisieren.

Daher liegt der vorliegenden Erfindung zum einen die Aufgabe zugrunde, DNA-Enzyme vom Typ 10-23 bereitzustellen, die eine größere Stabilität gegenüber nukleolytischem Abbau aufweisen, wobei die katalytische Aktivität gegenüber dem jeweiligen RNA-Substrat im wesentlichen derjenigen des nicht-stabilisierten DNA-Enzyms entspricht, vorzugsweise höher ist

Die effektive Behandlung von Schmerz ist eine große Herausforderung für die molekulare Medizin. Akuter und transitorischer Schmerz ist ein wichtiges Signal des Körpers, um den Menschen vor schwerem Schaden durch Umgebungseinflüsse oder eine Überlastung des Körpers zu schützen. Im Gegensatz dazu hat der chronische Schmerz, der länger anhält als die Ursache des Schmerzes und der erwartungsgemäße Zeitrahmen der Heilung, keine bekannte biologische Funktion. Vom chronischen Schmerz sind Hunderte von Millionen Menschen weltweit betroffen. Etwa 7,5 Millionen Menschen leiden allein in der Bundesrepublik Deutschland an chronischen Schmerzen. Die pharmakologische Behandlung, insbesondere des chronischen Schmerzes, ist derzeit unbefriedigend. Die im Stand der Technik bekannten Analgetika sind häufig nicht ausreichend wirksam und haben zum Teil schwere Nebenwirkungen.

Daher werden vielfach neue Targets, körpereigene Strukturen, über die eine schmerzmodulierende Einwirkung,bspw. niedermolekularer Wirkstoffe oder anderer Verbindungen wie Antisense-Oligodesoxyribonukleotide (ODN), möglich erscheint, für die Behandlung, insbesondere chronischer Schmerzen gesucht. Der von Caterina et al. (1997) Nature 389: 816-824, klonierte Vanilloid-Rezeptor 1 (VR1) (auch als Capsaicin-Rezeptor bezeichnet) ist ein vielversprechender Anhaltspunkt für die Entwicklung neuer Schmerzmedikamente. Es handelt sich bei diesem Rezeptor um einen Kationenkanal, der vorliegend von primären sensorischen Neuronen exprimiert wird (Caterina et al. (1997), *supra).* Der VR1 wird durch Capsaicin, eine Komponente der Chillischoten, Hitze (> 43°C) und einem niedrigen pH-Wert (d.h. Protonen) infolge von Gewebevertetzungen aktiviert und bewirkt einen Calcium-Einstrom in primäre afferente Neuronen. VR1-Knockout-Mäuse entwickeln keine thermische Hyperalgesie nach Gewebeschäden bzw. Entzündungen (Caterina et al. (2000) Science 288: 306-313; Davis et al. (2000) Nature 405: 183-187).

In WO 02/18407 sind Antisense-ODN und ebenfalls unter diesem Begriff subsummierte DNA-Enzyme vom Typ 10-23 offenbart, welche zur Spaltung der VR1-mRNA führen.

So zeigt bspw. Sioud et al. U. Mol. Biol. (2000), 296, 937-947) DNA-Enzyme des Typs 10-23, die in der katalytischen Kernsequenz und in den Substraterkennungsarmen Phosphorthioat-Nukleotide enthalten. Sioud *et al.* (2000) beschreibt jedoch keine DNA-Enzyme gegen den Vanilloid-Rezeptor-1 (VR-1). Ebenso zeigt Sioud *et al.* (2000) keinerlei Anwendungen von DNA-Enzymen bei der Behandlung von Schmerz oder anderen mit VR-1 zusammenhängenden krankhaften Zuständen.

Die WO 02/18407 beschreibt Antisense-Oligonukleotide gegen VR-1 sowie die Verwendung dieser Oligonukleotide in der Schmerztherapie. WO 02/18407 beschreibt jedoch ebenfalls keine DNA-Enzyme gegen den Vanilloid-Rezeptor-1 (VR-1) oder diskutiert im Allgemeinen Anwendungen von DNA-Enzymen bei der Behandlung von Schmerz oder anderen mit VR-1 zusammenhängenden krankhaften Zuständen.

Weiterhin zeigt Warashina et al. (Chemistry & Biology, April 1999, 6, 237-250) teilweise modifizierte DNA-Enzyme des Typs 10-23, die am 5'- und am 3'-Ende Phosphorthioat-Nukleotide bzw. 2'-O-MethylNukleotide enthalten. Wie auch bei Sioud *et al.* (2000) zeigen die DNA-Enzyme in Warashina *et al.* (1999) jedoch lediglich eine erhöhte Resistenz gegen Nukleasen, nicht jedoch eine erhöhte katalytische Aktivität. Auch beschreibt Warashina *et al.* (1999) keine DNA-Enzyme gegen den Vanilloid-Rezeptor-1 (VR-1) oder zeigt Anwendungen von DNA-Enzymen bei der Behandlung von Schmerz oder anderen mit VR-1 zusammenhängenden krankhaften Zuständen.

Daher liegt der vorliegenden Erfindung weiterhin die Aufgabe zugrunde, DNA-Enzyme vom Typ 10-23 bereitzustellen, die gegen die mRNA des VR1-Rezeptors gerichtet sind und eine größere Stabilität als die im Stand der Technik offenbarten ODN bei vergleichbarer oder höherer katalytischer Aktivität aufweisen.

Zu den Picomaviren gehören epidemiologisch bedeutsame Krankheitserreger, wie Rhinoviren, zahlreiche Enteroviren (u.a. Echoviren,die drei Polioviren, verschiedene Coxsackieviren), die unterschiedliche Krankheiten, insbesondere beim Menschen auslösen, wie Schnupfen (akute Rhinitis) oder schwerwiegenderen chronischen Entzündungen des Nasen-Rachenraums (Rhinopathien), Kinderlähmung entzündliche Herzerkrankungen, virale Meningitis usw.. Weitere Aufgabe der vorliegenden Erfindung ist daher, besonders wirksame und stabile DNA-Enzyme vom Typ 10-23 gegen Picomaviren bereitzustellen.

Die vorstehend genannten Aufgaben werden durch die in den Ansprüchen gekennzeichneten Ausführungsformen der vorliegenden Erfindung gelöst.

Insbesondere wird ein DNA-Enzym vom Typ 10-23 (im folgenden als "DNA-Enzym" bezeichnet) bereitgestellt, umfassend von 5' nach 3' einen ersten Substraterkennungsarm (im folgenden "Abschnitt I"), eine katalytische Kernsequenz (im folgenden "Abschnitt II") und einen zweiten Substräterkennungsarm (im folgenden "Abschnitt III"), wobei in Abschnitt II lediglich eines oder mehrere der Nukleotide 2, 7, 8 11, 14 und 15 (der vorzugsweise insgesamt 15 Nukleotide enthält) chemisch modifiziert ist/sind und wobei das/die modifizierte(n) Nukleotid(e) aus der Gruppe, bestehend aus Phosphorthioat-Nukleotiden, invertiertem Thymidin, 2'-O-Methyl-Ribonukleotiden und LNA-Ribonukleotiden, ausgewählt ist/sind.

Die vorliegende Erfindung beruht auf der überraschenden Feststellung, daß in geeigneter Weise chemisch modifizierte Nukleotide in DNA-Enzyme, insbesondere solche gegen VR1 oder Picomaviren, in der Kemsequenz an den Positionen 2, 7, 8, 11, 14 und/oder 15 eingeführt werden können sowie, wie nachstehend weiter dargelegt, auch in den Substraterkennungsarmen (Abschnitte I und III), gegebenenfalls unter Anpassung der Länge dieser Substraterkennungsarme, eingeführt werden können, um so stabilisierte DNA-Enzyme zu erhalten, die keine signifikante Verminderung der katalytischen Aktivität oder sogar eine verbesserte katalytische Aktivität gegenüber dem jeweiligen RNA-Substrat aufweisen.

Der Begriff "modifiziertes Nukleotid" bedeutet erfindungsgemäß, daß das jeweilige Nukleotid chemisch modifiziert ist. Ein Fachmann versteht unter dem Begriff "chemische Modifikation", daß das modifizierte Nukleotid durch Ersetzen, Anfügen oder Entfernen einzelner oder mehrerer Atome oder Atomgruppen im Vergleich zu natürlich vorkommenden Nukleotiden verändert ist. Daher kann die chemische Modifikation eines Nukleotids erfindungsgemäß die Ribose (z.B. 2'-O-Methyl-Ribonukleotide, sog. "Locked Nucleic Acid" (LNA)-Ribonukleotide und invertiertes Thymidin), die Phosphor(di)ester-Bindung (z.B. Phosphorthioate, Phosphoramidate, Methylphosphonate und Peptidnukleotide) und/oder die Base (z.B. 7-Deazaguanosin, 5-Methylcytosin und Inosin) betreffen.

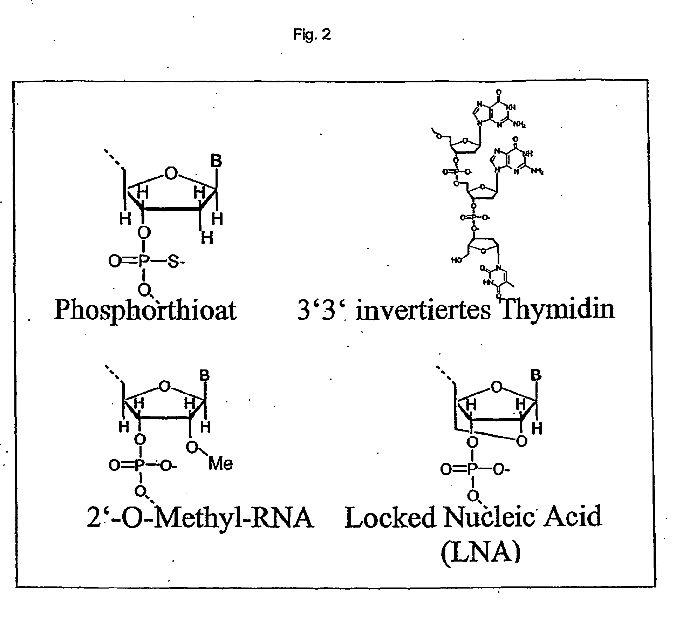

Bevorzugte modifizierte Nukleotide gemäß der vorliegenden Erfindung sind bspw. Phosphorthioat-Nukleotide, invertiertes Thymidin, 2'-O-Methyl-Ribonukleotide und LNA-Ribonukleotide. Diese erfindungsgemäßen Modifikationen sind beispielhaft in der Fig. 2 dargestellt.

Wie in der Fig. 2 gezeigt, sind LNA Ribonukleotide oder Desoxyribonukleotide, die eine Methylen-Brücke enthalten, welche den 2'-Sauerstoff der Ribose mit dem 4'-Kohlenstoff verbindet. Einen Überblick über LNA geben z.B. Braasch und Corey (2001) in Chem. Biol. 8: 1-7. LNA sind im Handel bspw. von der Fa. Proligo, Boulder, CO, USA,erhältlich.Phosphorthioate können bspw. über MWG-Biotech AG, E-bersberg, Deutschland, bezogen werden. 2'-O-Methyl-modifizierte Ribonukleotide sind u.a. bei IBA-NAPS, Göttingen, Deutschland, erhältlich.

Bei bevorzugten DNA-Enzymen der vorliegenden Erfindung sind alle der Nukleotide 2, 7, 8, 11, 14 und 15 des Abschnitts II (Kernsequenz) modifiziert, wobei das/die modifizierte(n) Nükleotid(e) aus der Gruppe, bestehend aus Phosphorthioat-Nukleotiden, invertiertem Thymidin, 2'-O-Methyl-Ribonukleotiden und LNA-Ribonukleotiden, ausgewählt ist/sind.

Einen weiteren Gegenstand der vorliegenden Erfindung bilden DNA-Enzyme, bei denen zusätzlich eines oder mehrere der Nukleotide der Substraterkennungsdomänen (bzw. Substraterkennungsarme), also des Abschnitts I und/oder des Abschnitts III modifiziert sind, insbesondere durch Phosphorthioat, invertiertes Thymidin, 2'-O-Methyl-Ribose oder LNA-Ribonukleotide, ist bzw. sind. Bevorzugte Ausführungsformen der vorliegenden Erfindung sind DNA-Enzyme, bei denen die Modifikationen der obigen Definition im Abschnitt II (katalytische Kernsequenz) und in den Abschnitten I und/oder II vorliegen.

Bei weiteren bevorzugten Ausführungsformen des erfindungsgemäßen DNA-Enzyms sind alle Nukleotide des Abschnitts I und/oder III, insbesondere alle Nukleotide beider Abschnitte, modifiziert, wobei es weiter bevorzugt ist, wenn dabei alle Nukleotide Phosphorthioat- oder 2'-O-Methyl-Ribose-modifiziert sind.

Des weiteren sind vorzugsweise mehr als 3, insbesondere 3 bis 7, mehr bevorzugt 3 bis 6, insbesondere 3 bis 5 Nukleotide des Abschnitts I und/oder des Abschnitts 3, vorzugsweise beider Abschnitte, modifiziert. Dabei ergeben sich be-sonders bevorzugte Ausgestaltungen des erfindungsgemäßen DNA-Enzyms, wenn sich die modifizierten Nukleotide am 5'-Ende des Abschnitts I und/oder am 3'-Ende des Abschnitts III, d.h. an den Enden des DNA-Enzyms, befinden. Hierbei sind die modifizierten Nukleotide vorzugsweise 2'-O-Methyl- oder LNA-Ribonükleotide.

2'-O-Methyl- und LNA-Ribonukleotide sind besonders bevorzugte Modifikationen gemäß der vorliegenden Erfindung, da diese Nukleotide eine höhere Affinität des DNA-Enzyms zum Substrat bewirken. Insbesondere bei Substratüberschuss (d.h. sog. "Multiple Tumover"-Bedingungen), wie sie für die Wirksamkeit des erfindungsgemäßen DNA-Enzyms unter *in vivo* Bedingungen anzutreffen sind, sollte die Affinität des Enzyms zum Substrat nicht zu hoch sein, da sonst die Produktfreisetzung die Kinetik bestimmt. Ein Maß für die Affinität des erfindungsgemäßen DNA-Enzyms ist sein Schmelzpunkt mit dem Substrat. Erfindungsgemäß wurde diesbezüglich festgestellt, daß ein Geschwindigkeitsoptimum beobachtet wird, welches z.B. bei gegen VR1 und Rhinovirus 14 gerichtete DNA-Enzymen etwas oberhalb der Reaktionstemperatur (37°C) liegt. Daher ergeben sich erfindungsgemäß besonders bevorzugte Ausgestaltungen des DNA-Enzyms, wenn bei einem gegebenen Ziel-Molekül die Schmelztemperatur durch Variation der modifizierten Nukleotide, der Art der Modifikation und/oder der Länge der Substraterkennungsarme (Abschnitte I und III) entsprechend eingestellt wird. Vorzugsweise beträgt die Schmelztemperatur der zwischen den Abschnitten I und III des erfindungsgemäßen DNA-Enzyms und den entsprechenden Targetsequenzen gebildeten Doppelstränge etwa 33 bis etwa 45°C, mehr bevorzugt etwa 35 bis etwa 42°C, insbesondere etwa 37 bis etwa 40°C, besonders etwa 39°C.

Dabei wurde insbesondere festgestellt, daß sich bevorzugte Ausführungsformen des erfindungsgemäßen DNA-Enzyms ergeben, wenn der Abschnitt I und/oder der Abschnitt III, insbesondere beide Abschnitte, nicht mehr als 8, insbesondere 7, Nukleotide umfassen.

Ganz besonders bevorzugte DNA-Enzyme sind Spezies mit folgenden Längen der Substraterkennungsarme und den folgenden Modifikationen, wobei die erste Zahl die Länge der Substraterkennungsarme und die zweite Zahl die Anzahl der modifizierten Nukleotide, die sich bevorzugt am Ende der Arme befinden, anzeigt und OMe 2'-O-Methyl-Ribonukleotide und LH LNA-Ribonukleotide kennzeichnen:
OMe9-4, OMe8-4, OMe7-3, OMe7-4, OMe7-5, OMe7-6, OMe7-7, OMe6-5, LH9-4, LH7-3 und LH7-4.

Die Basensequenz der katalytisch aktiven Kerndomäne des DNA-Enzyms, welches von Santoro und Joyce (1997), *supra,* entwickelt wurde, ist von 5' nach 3' GGCTAGCTACAACGA (vgl. Fig. 1). Erfindungsgemäß wurde weiter festgestellt, daß die Basen der Kemsequenz flexibel sind, insbesondere in den Positionen 7 bis 12, wobei das Thymidin an Position 8 sogar weggelassen werden kann. Aus diesen Erkenntnissen ergibt sich, daß das DNA-Enzym im Abschnitt II von 5' nach 3' die folgende Konsensussequenz aufweisen kann: GGMTMGH (N) DNNNMGD.

Dabei ist M = A oder C, H = A, C oder T, D = G, A oder T und N = irgendeine (natürlich vorkommende) Base. Basen bzw. Nukleotide in Klammem müssen nicht vorhanden sein.

Hinsichtlich des Substrats ist das erfindungsgemäße DNA-Enzym nicht eingeschränkt. Daher kann das DNA-Enzym grundsätzlich gegen sämtliche mRNA-Moleküle, andere RNA, wie virale RNA, Viroide usw. eingesetzt werden. Target-Sequenzen sind dabei solche, welche das Spaltmotiv der 10-23-DNA-Enzyme, nämlich ein Purin/Pyrimidin-Motiv, aufweisen. Bevorzugte Target-Sequenzen umfassen ein GU-Motiv, da GU-Sequenzen von DNA-Enzymen besonders effektiv gespalten werden.

Bevorzugt ist das erfindungsgemäße DNA-Enzym gegen die mRNA des Vanilloid-Rezeptors 1 (VR1), insbesondere von Säugern, wie Mensch, Affe, Hund, Katze, Ratte, Maus, Kaninchen, Meerschweinchen, Hamster, Rind, Schwein, Schaf und Ziege, gerichtet.

Spezifische-Basensequenzen der Abschnitte I und III (von 5' nach 3') sind die folgenden, wobei gegebenenfalls auch eine in einem Nukleotid davon abweichende Sequenz möglich ist, mit der Maßgabe, daß sich das von den angegebenen Sequenzen abweichende Nukleotid nicht an einer der letzten drei Positionen des Abschnitts I und nicht an einer der ersten 3 Positionen des Abschnitts III befindet, sind folgende:(N= irgendeine Base bzw.irgendein Nukleotid)

| Abschnitt I | Abschnitt III |
|---|---|
| GTCATGA | GGTTAGG |
| TGTCATGA | GGTTAGGG |
| ATGTCATGA | GGTTAGGGG |
| GTCGTGG | GATTAGG |
| TGTCGTGG | GATTAGG |
| ATGTCGTGG | GATTAGG |
| TTGTTGA | GGTCTCA |
| CTTGTTGA | GGTCTCAC |
| TCTTGTTGA | GGTCTCACC |
| TTGTTGA | AGTCTCA |
| CTTGTTGA | AGTCTCAN |
| TCTTGTTGA | AGTCTCANN |
| GGCCTGA | CTCAGGG |
| CGGCCTGA | CTCAGGGA |
| TCGGCCTGA | CTCAGGGAG |
| TGCTTGA | CGCAGGG |
| CTGCTTGA | CGCAGGGN |
| TCTGCTTGA | CGCAGGGNN |
| GTGTGGA | TCCATAG |
| GGTGTGGA | TCCATAGG |
| TGGTGTGGA | TCCATAGGC |
| ACGTGGA | TCAGACG |
| GACGTGGA | TCAGACGN |
| CGACGTGGA | TCAGACGNN |
| GTGGGGA | TCAGACT |
| GGTGGGGA | TCAGACTC |
| GGGTGGGGA | TCAGACTCC |
| | |
| GTGGGTC | GCAGCAG |
| AGTGGGTC | GCAGCAG |
| GAGTGGGTC | GCAGCAG |
| CGCTTGA | AAATCTG |
| GCGCTTGA | AAATCTGT |
| TGCGCTTGA | AAATCTGTC |
| CGCTTGA | GAATCTG |
| GCGCTTGA | GAATCTGN |
| TGCGCTTGA | GAATCTGNN |
| CTCCAGA | ATGTGGA |
| GCTCCAGA | ATGTGGAA |
| AGCTCCAGA | ATGTGGAAT |
| CTCCAGG | AGGTGGA |
| GCTCCAGG | AGGTGGA |
| AGCTCCAGG | AGGTGGA |
| GGTACGA | TCCTGGT |
| GGGTACGA | TCCTGGTA |
| CGGGTACGA | TCCTGGTAG |
| GGTGCGG | TCTTGGC |
| GGGTGCGG | TCTTGGC |
| CGGGTGCGG | TCTTGGC |

Weitere bevorzugte DNA-Enzyme der vorliegenden Erfindung sind gegen Virus-RNA, insbesondere gegen einen Picomavirus, wie bspw. in Kayser et al., Medizinsische Mikrobiologie, 8. Aufl., Thieme Verlag, Stuttgart, 1993, offenbart (z.B. Hepatitis-A-Virus, humane Enteroviren, wie Polio-, Coxsackie- und Echoviren, tierische Enteroviren, wie das für Schweine pathogene Tschen-Virus, humane Rhinoviren, wie Rhinovirus 14 usw. (ein Fachmann kennt über 80 Typen), tierische Rhinoviren, wie das Mauhund-Klauensäuche (MKS)-Virus, und Caliciviren, wie das Virus des vesikulären Exanthems der Schweine), gerichtet.

Die bevorzugte Ziel-RNA ist bspw. vom (vorzugsweise humanen) Rhinovirus 14 abgeleitet, wobei besonders vorteilhafte Ausführungsformen des DNA Enzyms der vorliegenden Erfindung in den Abschnitten I und III zur 5'-nicht-translatierten Region (5'-UTR) mindestens teilweise komplementär sind. Dieser Bereich umfasst eine Konsensussequenz, die bei zahlreichen Picomaviren mit einer Gruppe I IRES konserviert ist. Spezifische Beispiele erfindungsgemäßer, gegen den humanen Rhinovirus 14 und andere Picomaviren, insbesondere solche mit einer Gruppe I IRES, gerichteter Sequenzen sind nachfolgend von 5' nach 3' angegeben, wobei gegebenenfalls auch eine in einem Nukleotid davon abweichende Sequenz möglich ist, mit der Maßgabe, daß sich das von den angegebenen Sequenzen abweichende Nukleotid nicht an einer der letzten drei Positionen des Abschnitts I und nicht an einer der ersten 3 Positionen des Abschnitts III befindet (N = irgendeine Base bzw. irgendein Nukleosid)

| Abschnitt I | Abschnitt III |
|---|---|
| GTGGGA | TTTAAGG |
| GGTGGGA | TTTAAGGA |
| GGGTGGGA | TTTAAGGAA |

Erfindungsgemäß wurde überraschenderweise festgestellt, dass die erfindungsgemäßen Gegenstände die Expression des VR1 stärker unterdrücken als gewöhnliche Antisense-Oligonukleotide.

Ein weiterer Gegenstand der vorliegenden Erfindung betrifft Wirtszellen, ausgenommen humane Keimzellen und humane embryonale Stammzellen, die mit einem mindestens einem erfindungsgemäßen DNA-Enzym transformiert sind. Erfindungsgemäße DNA-Enzyme können in die jeweilige Wirtszelle über übliche Methoden, bspw. Transformation, Transfektion, Transduktion, Elektroporation oder Partikel-Gun eingebracht werden. Als Wirtszellen kommen alle Zellen pro- oder eukaryotischer Natur in Betracht, bspw. von Bakterien, Pilzen, Hefen, pflanzlichen oder tierischen Zellen. Bevorzugte Wirtszellen sind Bakterienzellen, wie E-*scherichia coli, Streptomyces, Bacillus* oder *Pseudomonas,* eukaryotische Mikroorganismen, wie *Aspergillus* oder *Saccharomyces cerevisiae* oder die gewöhnliche Bäckerhefe (Stinchcomb et al. (1997) Nature 282: 39).

In einer bevorzugten Ausführungsform werden jedoch zur Transformation mittels erfindungsgemäßer DNA-Enzyme Zellen aus multizellulären Organismen gewählt. Im Prinzip ist jede höhere eukaryotische Zellkultur als Wirtszelle verfügbar, wenn auch Zellen von Säugern, bspw. Affen, Ratten, Hamstem, Mäusen oder Menschen, ganz besonders bevorzugt sind. Dem Fachmann ist eine Vielzahl von etablierten Zellinien bekannt. In einer keineswegs abschlieβenden Aufzählung werden die folgenden Zellinien genannt: 293T (Embryonennierenzellinie) (Graham et al., J. Gen. Virol. 36: 59 (1997), BHK (Babyhamstemierenzellen), CHO (Zellen aus den Hamsterovarien, Urlaub und Chasin, Proc. Natl. Accad. Sci. USA 77: 4216, (1980)), HeLa (humane Karzinomzellen) und weitere - insbesondere für den Laboreinsatz etablierte - Zellinien, bspw. HEK293-, SF9-oder COS-Zellen. Ganz besonders bevorzugt sind humane Zellen, insbesondere neuronale Stammzellen und Zellen des "Schmerz-Weges", vorzugsweise primäre sensorische Neuronen. Humane Zellen, insbesondere autologe Zellen eines Patienten, eignen sich nach (vor allem *ex vivo)* Transformation mit erfindungsgemäßen DNA-Enzymen, ganz besonders als Arzneimittel für bspw. gentherapeutische Zwecke, also nach Durchführung einer Zellentnahme, ggf. *ex vivo* Expansion, Transformation, Selektion und abschließender Retransplantation in den Patienten.

Die Kombination aus einer Wirtszelle und einem zur Wirtszelle passenden erfindungsgemäßen DNA-Enzym bildet ein System, das zur Anwendung der erfindungsgemäßen DNA-Enzyme dienen kann.

Somit eignen sich die erfindungsgemäßen Gegenstände als Arzneimittel, bspw. zur Inhibition der Nozizeption, bspw. aufgrund der Verminderung der Expression des VR1-Rezeptors mittels erfindungsgemäßer DNA-Enzyme.

Folglich umfaßt die vorliegende Erfindung auch die Verwendung der vorstehend genannten Gegenstände zur Behandlung bzw. zur Herstellung eines Arzneimittels zur Behandlung und/oder Prävention von Schmerzen, insbesondere akuten oder chronischen Schmerzen, sowie auch die Verwendung zur Behandlung bzw. zur Herstellung eines Arzneimittels zur Behandlung von Sensibilitätsstörungen, die mit dem VR1-Rezeptor zusammenhängen, bspw. zur Behandlung von Hyperalgesien, Neuralgien und Myalgien, sowie von allen mit VR1 zusammenhängenden Erkrankungen und Erkrankungssyomptomen, insbesondere Harninkontinenz, neurogenen Blasensymptomen, Pruritus, Tumoren und Entzündungen.

Erfindungsgemäße Arzneimittel bzw. unter Verwendung der erfindungsgemäßen Gegenstände hergestellte Arzneimittel enthaltend neben den vorstehend definierten Gegenständen ggf. einen oder mehrere geeignete Hilfs- und/oder Zusatzstoffe. Erfindungsgemäße Arzneimittel können als flüssige Arzneiform in Form von Injektionslösung, Tropfen oder Säfte, als halbfeste Arzneiformen in Form von Granulaten, Tabletten, Pellets, Patches, Kapseln, Pflaster oder Aerosolen verabreicht werden und enthalten neben dem mindestens einen erfindungsgemäßen Gegenstand je nach galenischer Form ggf. Trägermaterialien, Füllstoffe, Lösungsmittel, Verdünnungsmittel, Farbstoffe und/oder Bindemittel. Die Auswahl der Hilfsstoffe sowie die einzusetzenden Mengen derselben hängen davon ab, ob das Arzneimittel oral, peroral, parenteral, intravenös, intraperitoneal, intradermal, intramuskulär, intranasal, bukkal, rektal oder topisch, den Schleimhäuten, den Augen usw., appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Gegenstände in einem Depot in gelöster Form oder in einem Pflaster, ggf. unter Zusatz von die Hautpenetration fördernden Mitteln sind geeignete perkutane Applikationszubereitungen. Oral oder perkutan anwendbare Zubereitungsformen können die erfindungsgemäßen Gegenstände verzögert freisetzen. Die einem Patienten zu verabreichende Wirkstoffmenge variiert in Abhängigkeit vom Gewicht des Patienten, von der Applikationsart, der Indikation und dem Grad der Erkrankung. Üblicherweise werden 2 bis 500 mg/kg Körpergewicht wenigstens eines erfindungsgemäßen Gegenstandes appliziert. Wenn das Arzneimittel insbesondere zur Gentherapie verwendet werden soll, empfehlen sich als geeignete Hilfs- oder Zusatzstoffe bspw. eine physiologische Kochsalzlösung, Stabilisatoren, Protease oder DNAse-Ihibitoren usw.

Geeignete Zusatz- und/oder Hilfsstoffe sind als bspw. Lipide, kationische Lipide, Polymere, Liposomen, Nukleinsäure-Aptamere, Peptide und Proteine (z.B. tet-, Transportin, Transferrin, Albumin oder Ferritin), vorzugsweise solche, die an DNA oder RNA gebunden sind (oder synthetische Peptid-DNA-Moleküle), um bspw. die Einbringung von Nukleinsäuren in die Zelle zu erhöhen, um das Arzneimittelgemisch auf nur eine Untergruppe von Zellen zu richten, um den Abbau der erfindungsgemäßen Nukleinsäure in der Zelle zu verhindern, um die Lagerung des Arzneimittelgemischs vor der Verwendung zu erleichtern usw. Beispiele für Peptide und Proteine oder synthetische Peptid-DNA-Moleküle sind z.B. Antikörper, Antikörperfragmente, Liganden, Adhäsionsmoleküle, die alle modifiziert oder unmodifiziert sein können. Hilfsstoffe, die bspw die DNA-Enzyme in der Zelle stabilisieren, sind z.B. Nukleinsäure-kondensierende Substanzen wie kationische Polymere, Poly-L-Lysin oder Polyethylenimin.

Bei einer lokalen Anwendung erfindungsgemäßer Gegenstände, bspw. von erfindungsgemäßen DNA-Enzymen erfolgt die Verabreichung durch Injektion, Katheter, Suppositorium ("Zäpfchen"), Aerosole (Nasen- bzw. Mundspray, Inhalation), Trokars, Projektile, pluronische Gele, Polymere, die anhaltend Medikamente freisetzen, oder irgendeine andere Vorrichtung, die einen lokalen Zugang ermöglicht. Auch die *ex vivo* Anwendung des erfindungsgemäßen Arzneimittelgemischs, das zur Behandlung der vorstehend genannten Indikationen verwendet wird, erlaubt einen lokalen Zugang.

Erfindungsgemäße Gegenstände können ggf. in einer Zusammensetzung als Arzneimittel- (Wirkstoff)gemisch mit bspw. mindestens einem weiteren Schmerzmittel oder antiviralem und/oder anderem zur Behandlung von mit (rhino-)viralen Infektionen in Zusammenhang stehenden Erkrankungen geeigneten Mittel kombiniert werden.

Auf diese Weise können erfindungsgemäße Gegenstände bspw. in Verbindung mit Opiaten und/oder synthetischen Opioiden (bspw. Morphin, Levomethadon, Codein, Tramadol, Bupremorphin) und/oder NSAID (bspw. Diclofenac, Ibuprofen, Paracetamol) kombiniert werden, bspw. in einer der vorgenannt offenbarten Verabreichungsformen oder auch im Zuge einer kombinierten Therapie in jeweils separierten Verabreichungsformen mit ggf. unterschiedlicher Konfektionierung in einem an die Bedürfnisse des jeweiligen Patienten angepassten medizinisch sinnvoll gestalteten Therapieplan. Bevorzugt ist die Verwendung derartiger Zusammensetzungen als Arzneimittelgemische mit bspw. etablierten Analgetika zur Behandlung (bzw. zur Herstellung von Arzneimitteln zur Behandlung) von vorliegend offenbarten medizinischen Indikationen.

Die erfindungsgemäßen DNA-Enzyme können nach einem Fachmann bekannten Verfahren hergestellt werden. Die entsprechenden Nukleotide werden bspw. nach Art der Merrifield-Synthese an einem unlöslichen Träger (H.G. Gassen et al. (1982) Chemical and Enzymatic Synthesis of Genefragments, Verlag Chemie, Weinheim) oder auf andere Art synthetisiert (Beyer und Walter (1984) Lehrbuch der organischen Chemie, Seite 816 ff., 20. Auflage, S. Hirzel Verlag, Stuttgart). Ebenfalls können die erfindungsgemäßen Gegenstände auch nach bekannten Verfahren *in situ* auf Glas, Kunststoff, oder Metall, z.B. Gold, synthetisiert werden.

Ein weiterer Gegenstand der vorliegenden Erfindung betrifft ein *ex vivo* Verfahren zur inhibition der Expression eines Gens, umfassend das Einbringen des vorstehend definierten DNA-Enzyms in eine das jeweilige Gen exprimierende Zelle. Ein bevorzugtes Zielgen des erfindungsgemäßen Verfahrens ist das VR1-Gen, wobei entsprechend ein gegen die mRNA des VR1 gerichtetes DNA-Enzym in die Zelle eingebracht werden bzw. wird.

Das Einbringen der erfindungsgemäßen Gegenstände in die Zelle kann dabei auf die vorstehend dargelegte Art und Weise erfolgen.

Die Figuren zeigen:
- Fig.1: ist die schematische Darstellung eines DNA-Enzyms des Typs 10-23 gemäß Santoro et al. (1997), *supra,* (Fig. 2, S. 4264) (einschließlich RNA-Substrat). Der obere, mit einem Pfeil gekennzeichnete Strang ist der zu spaltende RNA-Strang, wobei der Pfeil die Spaltstelle angibt. Der untere Strang ist eine Darstellung des DNA-Enzyms. In Bezug auf bevorzugte Ausführungsformen des erfindungsgemäßen DNA-Enzyms ist dabei im oberen Strang das Y = U und das R = G. Die Spaltstelle auf dem oberen Strang ist daher eine sog. GU-Spaltstelle, die von DNA-Enzymen besonders effektiv gespalten wird. Entsprechend ist R im unteren Strang = A. Dem schließen sich 5'-wärts bspw. die weiteren Nukleotide aus den vorstehend definierten Abschnitten I an. Im Abschnitt III schließen sich dann direkt an das am Abschnitt III anliegend ungepaarte A aus 5'-Richtung 3'-wärts die Nukleotide des zweiten zum RNA-Substrat komplementären Teils des DNA-Enzyms, bspw. die vorstehend genannten Sequenzen, an. Die Abschnitte I und Abschnitte III binden also das Substrat und werden daher als. Substraterkennungsarme des DNA-Enzyms bezeichnet
- Fig. 2: zeigt die Strukturen von Phosphorthioat-Nukleotiden, inverbiertern Thymidin, 2'-O-Methyl-Ribonukleotiden und LNA-Ribonukleotiden.

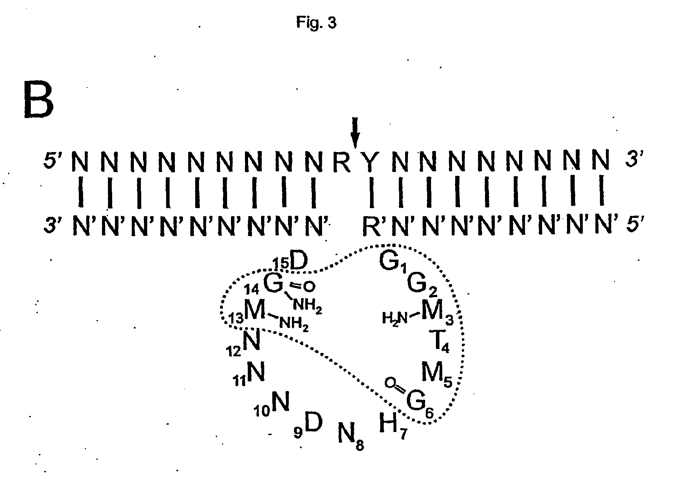

- Fig. 3: zeigt in einer der Fig. 1 entsprechenden Darstellung die allgemeine Struktur eines DNA-Enzyms vom Typ 10-23 mit der erfindungsgemäßen Konsensussequenz des Abschnitts II (katalytische Kemsequenz). Nicht-essentielle Nukleotide, die durch jedes andere Nukleotid ohne wesentlichen Aktivitätsverlust ausgetauscht werden können, sind mit N bezeichnet, N' steht für jedes zu N komplementäre Nukleotid. R bezeichnet ein Nukleotid, das bevorzugt eine Purin-Base enthält.Y steht für ein Nukleotid,das bevorzugt eine Pyrimidin-Base enthält. R' steht für ein zu Y komplementäres Nukleotid, das ein Purin enthält. M steht für A oder C, H steht für A, C oder T, und D steht für G, A oder T. Der Bereich, der wahrscheinlich direkt an der Bildung der katalytischen Zentrums beteiligt ist, ist mit einer gepunkteten Linie markiert. Exocyclische funktionelle Gruppen, die zur Aktivität des DNA-Enzyms erforderlich sind, sind jeweils angegeben.

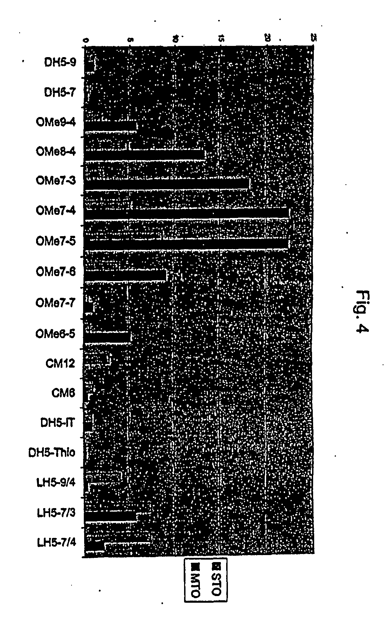

- Fig. 4: zeigt in einer graphischen Darstellung die relativen Aktivitäten modifizierter DNA-Enzyme gegen die 5'-UTR des humanen Rhinovirus 14 (DH 5) jeweils unter 10fachem Überschuß an DNA-Enzymen ("Single Tumover"-Bedingungen, STO, jeweils linker Balken) und 10fachem Substratüberschuß ("Multiple Tumover"-Bedingungen, MTO, jeweils rechter Balken). Die Aktivitäten sind auf das unmodifizierte DNA-Enzym mit 9 Nukleotiden langen Substraterkennungsarmen normiert. Die angegebenen DNA-Enzyme wiesen die folgenden Modifikationen auf: Phosphorthioat (Thio), invertiertes Thymidin (iT), 2'-O-Methyl-RNA (OMe) und LNA (LH). Die erste Zahl bedeutet dabei die Länge der Substraterkennungsarme, während die zweite Zahl die Anzahl der modifizierten Nukleotide am Ende der Arme angibt.CM6 ist ein erfindungsgemäßes DNA-Enzym, bei dem die Nukleotide 2, 7, 8, 11, 14 und 15 der Kemsequenz modifiziert sind (2'-O-Methyl-Ribonukleotide). CM12 ist ein Vergieichskonstrukt, bei dem 12 Nukleotide der Kemsequenz (alle außer Positionen 3, 5 und 10) modifiziert sind. DH5-9 bezeichnet das unmodifizierte DNA-Enzym gegen die 5'-UTR des humanen Rhinovirus 14 mit 9 Nukleotiden langen Substraterkennungsarmen, während DH5-7 das entsprechende DNA-Enzym mit 7 Nukleotiden langen Substraterkennungsarmen kennzeichnet. Durch optimales Design der Substraterkennungsarme (siehe OMe7-4 und OMe7-5) kann die Aktivität des DNA-Enzyms unter MTO-Bedingungen, die z.B. für den Einsatz *in vivo* entscheidend sind, um mehr als den Faktor 20 erhöht werden. Das Enzym, bei dem die genannten 6 Nukleotide in der Kernsequenz zusammen gegen die entsprechenden 2'-O-Methyl-Ribonukleotide ausgetauscht sind, ist noch immer im wesentlichen so aktiv wie das unmodifizierte DNA-Enzym (vgl. CM6 und DH 5-9). Das DNA-Enzym, bei dem 12 Nukleotide der Kemsequenz modifiziert sind, spaltet unter STO-Bedingungen die vollständige 5'-UTR mit guter Effektivität, ist jedoch unter MTO-Bedingungen gegenüber dem vollständigen (langen) Target inaktiv (vgl. CM12). Werden die in CM12 gemeinsam ausgetauschten Nukleotide jeweils einzeln modifiziert, spalten die entsprechenden Konstrukte einen 19-meren kurzen Abschnitt aus der Ziel-RNA (5'-UTR des humanen Rhinovirus 14), der zu den Substraterkennungsarmen komplementär ist (nicht gezeigt). Die Balken zeigen jeweils den Mittelwert dreier unabhängiger Experimente.

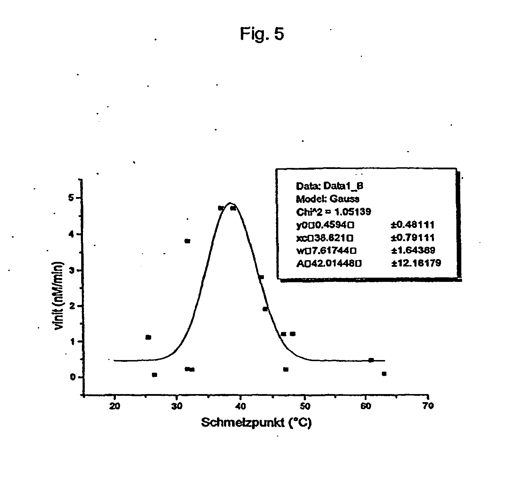

- Fig. 5: zeigt in einem Diagramm die Abhängigkeit der Anfangsgeschwindigkeit der Substratspaltung (vᵢₙᵢₜ) vom Schmelzpunkt der bei einem DNA-Enzym gebildeten Helices zwischen den Abschnitten I bzw. III (Substraterkennungsarme) mit der Targetsequenz im Fall der gemäß Fig. 4 untersuchten DNA-Enzyme. Danach ist ein Optimum der Anfangsgeschwindigkeit vom Schmelzpunkt, das etwas oberhalb der Reaktionstemperatur (37°C) liegt, festzustellen. Im vorliegenden Fall ergibt sich eine optimale Schmelztemperatur von 39°C.

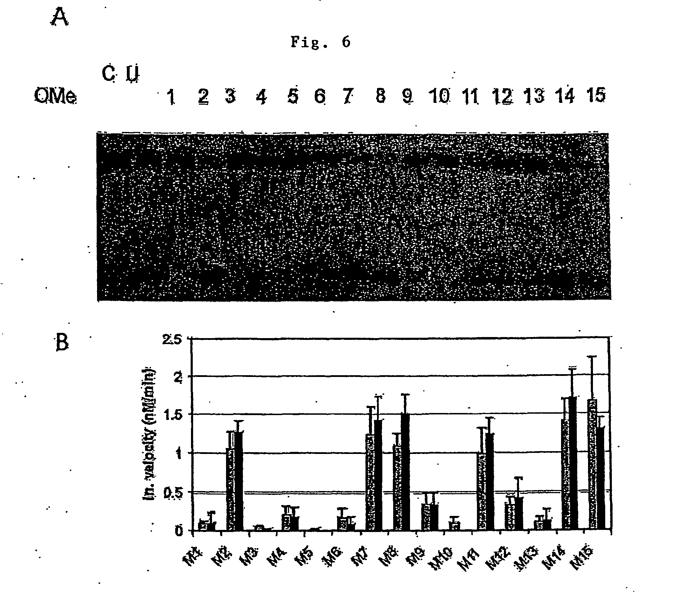

- Fig.6: zeigt in Figur 6A die Ergebnisse eines Experiments auf der Basis eines hundertfachen Überschusses der kurzen 19 Nukleotide langen Ziel-RNA (target RNA). Hierzu wurde dieser Überschuß mit DNAzymen bei 37°C für 20 min inkubiert. Das ungespaltene Substrat (obere Bande) und das 5'-Spaltprodukt (untere Bande) sind zu sehen. Folgende Auftragungen sind in den einzelnen Spuren zu sehen: Spur 1: Kontrolle ohne Enzym, Spur 2: nicht modifiziertes DH5, Spuren 3 bis 17 (bezeichnet als 1 bis 15 in der Auftragung): DNAzyme mit 2'-O-Methyl-RNA jeweils an einer der Positionen 1 bis 15 (in entsprechender numerischer Reihenfolge). Figur 6B zeigt in einer graphischen Darstellung die relativen Spaltungsaktivitäten von DNA-Enzymen gegen die 5'-UTR des humanen Rhinovirus 14, bei denen jeweils ein Nukleotid der Kemsequenz (Positionen 1 bis 15 entsprechend den Proben M1 bis M15) einzeln durch 2'-O-Methyl-Modifikationen modifiziert wurden. Die jeweilige Aktivität ist auf das entsprechende unmodifizierte DNA-Enzym mit 9 Nukleotiden langen Erkennungsarmen normiert. Danach können die Nukleotide 2, 7, 8, 11, 14 und 15 ohne Aktivitätsverlust bzw. mit Aktivitätsgewinn modifiziert werden. Die Balken zeigen jeweils den Mittelwert dreier unabhängiger Experimente mit der angegebenen Standardabweichung, und zwar für die kurzen Zielmoleküle (graue Balken) und die langen Zielmoleküle (schwarze Balken). Die Balkenhöhe gibt den Prozentsatz der Spaltung von Ziel-RNA durch DNAzyme (DNA-Enzyme) an.

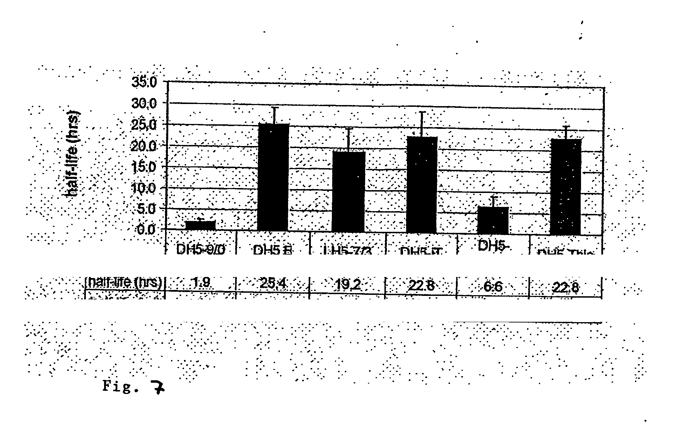

- Fig. 7: In Figur 7 ist die Stabilität von modifizierten DNA-Enzymen im Zellkulturmedium dargestellt. Hierzu wurden die DNA-Enzyme bei einer Endkonzentration von 1µM in DMEM mit 10%igem fötalen Rinderserum inkubiert. Aliquots wurden zu bestimmten Zeiten entnommen und die verbleibende Menge von Volllängenoligonukleotiden wurde durch Polyacrylamid-Gelelektrophorese bestimmt. Durchschnittliche Halbwertszeiten und Halbwertszeiten ("half life"), normalisiert gegenüber nicht-modifiziertem DNAzym aus drei Experimenten werden angegeben. Die Bedeutungen der Abkürzungen sind in der Tabelle 2 angegeben.
Modifikationen der Bindungsarme mit LNA-Nukleotiden und Phophorthionaten erhöhte die Halbwertszeit von 2h auf ca. mehr als 20 h. Ein invertiertes Thymidin am 3'-Ende erhöhte die Stabilität um den Faktor 10. 2'-O-Methylmodifikationen, die ausschließlich an den Bindungsarmen angeordnet sind, erwiesen sich als anderen Modifikationen deutlich unterlegen (Halbwertszeiten von ca. 6,5 h). Das neu entworfene DNAzym DH5 E (mit 2'-O-Methylnukleotiden an beiden Bindungsarmen und dem katalytischen Zentrum) erwies sich als gegenüber Degradation ausgesprochen resistent. Die Halbwertszeit wurde auf 25 h erhöht.

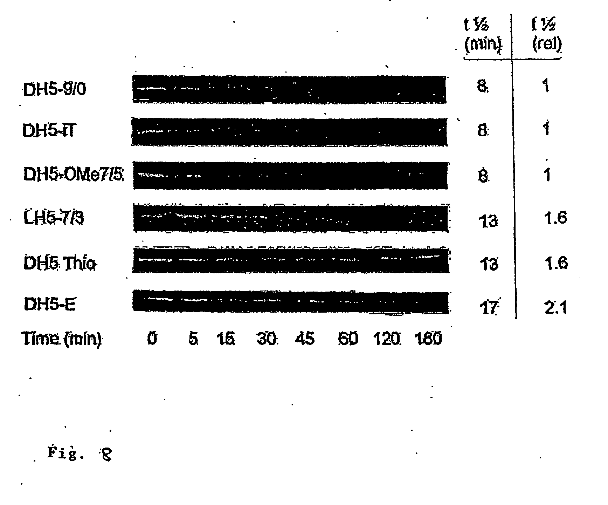

- Fig. 8: Figur 8 stellt einen Vergleich der Stabilität von DNAzymen gegenüber der S1-Endonuklease dar. DNAzyme wurden bei einer Konzentration von 2µM mit 0,4 U S1 Endonuklease inkubiert pro 100 pmol DNAzym inkubiert. Aliquots wurden zu entsprechenden zeitpunkten abgenommen. Volllängen- und abgebaute Oligonukleotide wurden auf einem 20%igen denaturierendem Polyacrylamidgel separiert. Halbwertszeiten und relative Stabilität, normalisiert gegenüber nicht-modifiziertem DNAzym sind angegeben.
Wie aus Figur 8 erkannt werden kann, wird unmodifiziertes DNA-zym und DNAzyme mit invertiertem Thymidin und 2'-O-Methyl RNA "end-blocks" fast vollständig nach 30 Minuten mit einer Halbwertszeit von ca. 8 Minuten abgebaut. Die Oligonukleotide, die LNA-Monomere enthalten, werden nur schwach durch Ethidiumbromid angefärbt. Es kann erkannt werden, daß das DNAzym, das durch die Einführung von LNAs in den Substraterkennungsarmen stabilisiert wurde, stabiler gegenüber S1-Endonuklease als das unmodifizierte DNAzym war. DH5-Thio zeigte eine zweiphasige Degradationskurve, was auf die chirale Natur der modifizierten Nukleotide zurückzuführen ist. Ungefähr 50% der Startmenge wird durch die Endonuklease mit einer Halbwertszeit von ca. 13 Minuten abgebaut. Die andere Hälfte unterliegt einer viel langsameren Degradationskonstante. Das optimierte DNAzym mit 2'-O-Methyl-Monomeren im katalytischen Kern und in den Substraterkennungsarmen hat die längste Halbwertszeit aller untersuchten modifizierten Enzyme. Seine Stabilität wird mehr als zweifach gegenüber dem unmodifizierten DNAzym erhöht.

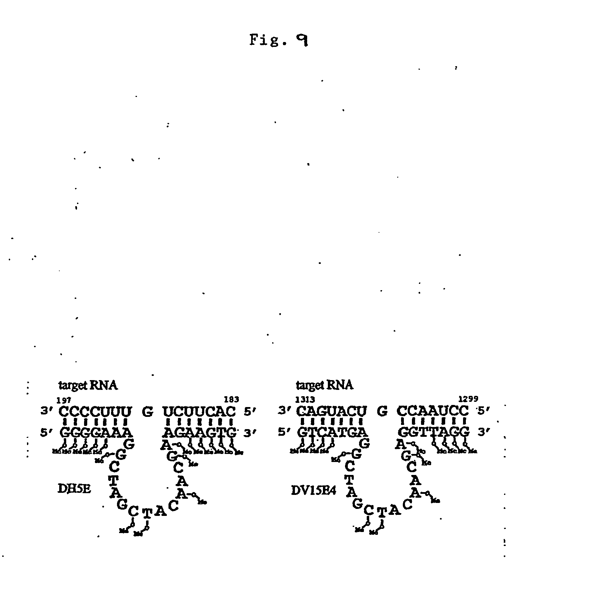

- Fig. 9: In Figur 9 werden die erfindungsgemäßen Modifikationen von optimierten DNAzymen dargestellt. Hierbei zeigt die linke Darstellung in Figur 9 DNAzyme gegen das 5'-NTR (nicht-translatierte Region) von HRV14 (DH5) und gegen VR1 mRNA (DV15) auf der rechten Seite und ihre jeweiligen Substrate RNAs. Die Position der Ziel-RNAs,die durch die DNAzyme gebunden sind, werden induziert und die Nukleotide, an welchen 2'-O-Methyl-RNA-Monomere in die DNA-zyme eingeführt wurden, sind markiert.

Die folgenden Ausführungsbeispiele erläutern die vorliegende Erfindung näher, ohne sie einzuschränken.

### Beispiel 1

### DNA-Enzyme

Es wurden DNA-Enzyme mit den folgenden Sequenzen untersucht:
1. DNA-Enzyme gegen VR1
   DV15-9: ATGTCGTGGGGCTAGCTACAACGAGATTAGG
   DV15-7: GTCGTGGGGCTAGCTACAACGAGATTAGG
   (Substraterkennungsarme unterstrichen)
   DV15-Konstrukte sind gegen das 15. GUC der mRNA des humanen VR1 gerichtet.
2. DNA-Enzyme gegen humanen Rhinovirus 14
   **DH5-9:** CCGGGGAAAGGCTAGCTACAACGAAGAAGTGCT
   **DH5-8:** CGGGGAAAGGCTAGCTACAACGAAGAAGTGC
   **DH5-7:** GGGGAAAGGCTAGCTACAACGAAGAAGTG
   (Substraterkennungsrme unterstrichen)
   DH5-Konstrukte sind gegen die 5'-UTR des humanen Rhinovirus 14, gerichtet, eine Konsensussequenz, die zwischen zahlreichen Picomaviren mit einer Gruppe I IHRES homolog ist.

*Substrate*
1. VR1 mRNA
   VR1 mRNA wurde durch *in vitro* Transkription hergestellt Zunächst wurde die cDNA des VR1 in den Vektor pcDNA3.1 (+) (Invitrogen) kloniert. Anschließend erfolgte die *in vitro* Transkription mit dem RiboMAX Large Scale RNA Production System - T7 (Promega) nach den Angaben des Herstellers.
2. 5'-UTR des humanen Rhinovirus 14
   Die der 5'-UTR des humanen Rhinovirus 14 entsprechende DNA wurde im Vektor pCR2.1 (Stratagene) von Prof. Zeichhardt (Universitätsklinikum Benjamin Franklin der Freien Universität Berlin) erhalten. Nach Linearisierung mit BamHI wurde zur Gewinnung der Ziel-RNA eine *in vitro* Transkription wie bei der VR1 mRNA durchgeführt.

### Messung von DNA-Enzymaktivitäten

Enzymaktivitäten wurden in 50 mM Tris-HCl pH 7,5 und 10 mM MgCl2 bei 37°C gemessen. Bei "Single Tumover" (STO)-Kinetiken wurden die DNA-Enzyme im 10-fachem Überschuß (100 nM RNA-Substrat; 1 µM DNA-Enzym) zum Substrat verwendet. Für "Multiple Turnover" (MTO)-Experimente wurde das RNA-Substrat im 10-fachen Überschuß (100 nM RNA-Substrat; 10 nM DNA-Enzym) eingesetzt. Es wurden jeweils Dreifach-Bestimmungen durchgeführt.

### Einfluß von Modifikationen in den Substraterkennungsarmen auf die Aktivität von DNA-Enzymen

Es wurden die Aktivitäten der unmodifizierten DNA-Enzyme DH-5 und DV-15 mit 9 Nukleotiden (DH5-9, DV15-9) bzw. 7 Nukleotiden (DH5-7) aufweisenden Substraterkennungsarmen mit folgenden in den Substraterkennungsarmen chemisch modifizierten DNA-Enzymen gleicher Basensequenz unter STO- und MTO-Bedingungen verglichen (Bei den 2'-O-Methyl-Ribose- und LNA-modifizierten Spezies befanden sich die modifizierten Nukleotide jeweils am 5'-Ende des ersten Substraterkennungsarms (Abschnitt I) und am 3'-Ende des zweiten Substraterkennungsarms (Abschnitt III).):

**Tab 1.: DNA-Enzyme mit spezifischen Nukleotid-Modifikationen in den Substraterkennungsarmen**

| Bezeichnung | Länge der Arme | Modifikation |
|---|---|---|
| OMe9-4 | 9 | 2'-O-Methyl-Ribose, 4 Nukleotide |
| OMe8-4 | 8 | 2'-O-Methyl-Ribose, 4 Nukleotide |
| OMe7-3 | 7 | 2'-O-Methyl-Ribose, 3 Nukleotide |
| OMe7-4 | 7 | 2'-O-Methyl-Ribose, 4 Nukleotide |
| OMe7-5 | 7 | 2'-O-Methyl-Ribose, 5 Nukleotide |
| OMe7-6 | 7 | 2'-O-Methyl-Ribose, 6 Nukleotide |
| OMe7-7 | 7 | 2'-O-Methyl-Ribose, 7 Nukleotide |
| OMe6-5 | 6 | 2'-O-Methyl-Ribose, 5 Nukleotide |
| DH5-iT | 9 | invertiertes Thymidin, 1 Nukleotid |
| DH5-Thio | 9 | Phosphorthioat, 9 Nukleotide |
| LH5-9/4 | 9 | LNA-Ribose, 4 Nukleotide |
| LH5-7/3 | 7 | LNA-Ribose, 4 Nukleotide |
| LH5-7/4 | 7 | LNA-Ribose, 4 Nukleotide |

Die Ergebnisse der Vergleichsexperimente mit den gegen humanen Rhinovirus 14 (d.h. DH5-Konstrukte) sind in der Fig. 4 dargestellt, wobei die Aktivität des unmodifizierten DNA-Enzyms mit 9 Nukleotiden langen Substraterkennungsarmen (DH5-9) gleich 1 gesetzt wurde. Die insbesondere für *in vivo* Anwendungen entscheidenden Aktivitäten unter MTO-Bedingungen zeigen, dass in den Substraterkennungsarmen modifizierte DNA-Enzyme keine wesentlichen Aktivitätseinbußen gegenüber den unmodifizierten Vergleichskonstrukten (DH5-9 und DH5-7) aufweisen. Darüber hinaus ist festzustellen, dass 2'-O-Methyl-Ribose- und LNAmodifizierte Konstrukte, insbesondere solche, bei denen nicht alle Nukleotide modifziert sind bzw. eine Anpassung der Länge der Substraterkennungsarme vorgenommen wird, eine gegenüber dem nicht modifizierten Vergleichskonstrukt zum Teil vervielfachte Aktivität zeigen. Bei 2'-O-Methyl-Ribose-modifizierten (3, 4 bzw. 5 modifzierte Nukleotide, Substraterkennungsarme mit 7 oder 8 Nukleotiden) ergeben sich Aktivitäten unter MTO-Bedingungen, die über 10fach bis über 20fach größer als diejenigen der unmodifizierten DNA-Enzyme sind.

Bei dem DNA-Enzym gegen VR1 (DV15) ergab sich für das Konstrukt mit 5 2'-O-Methyl-Ribonukleotiden an den Enden von 7 Nukleotide langen Substraterkennungsarmen im Vergleich zum unmodifizierten Konstrukt folgender Wert der Anfangsgeschwindigkeit unter MTO-Bedingungen:
- DV15 (unmod.):: 0,5 ± 0,1 nM/min
- DV15-7/5:: 1,7 ± 0,2 nM/min

Somit ist das modifizierte Konstrukt gegen den VR1 mehr als dreimal so aktiv wie das korrespondierende unmodifizierte DNA-Enzym.

### Beispiel 2

### Abhängigkeit der Reaktionsgeschwindigkeit vom Schmelzpunkt zwischen DNA-Enzym und Substrat

Die erfindungsgemäßen Modifikationen 2'-O-Methyl- und LNA-Ribonukleotide erhöhen die Affinität zum Substrat (d.h. der Schmelzpunkt der zwischen dem Abschnitt **I** bzw. III mit der Targetsequenz gebildeten Helices erhöht sich). Für eine optimale Aktivität unter MTO-Bindungen ist jedoch auch eine effiziente Produktfreisetzung erforderlich, weshalb die Affinität zum Substrat nicht zu hoch sein sollte. Wird daher bei den erfindungsgemäß modifizierten DNA-Enzymen gemäß vorstehender Tab. 1 die Anfangsgeschwindigkeit unter MTO-Bedingungen (vᵢₙᵢₜ) in Abhängigkeit von der Schmelztemperatur mit dem Substrat aufgetragen, so wird eine Korrelation zwischen der Schmelztemperatur und der Reaktionsgeschwindigkeit beobachtet, die in erster Näherung einer Gauss-Verteilung um ein Optimum bei etwa 39°C angeglichen werden kann (Fig. 5).

### Beispiel 3

### Einfluß von Modifikationen in der Kernsequenz auf die Aktivität von DNA-Enzymen

Die 15 Nukleotide der Kemsequenz von DH5 wurden einzeln durch entsprechende 2'-O-Methyl-Rbonukleofide ersetzt und die Reaktionsgeschwindigkeit unter MTO-Bedingungen, wie im Beispiel 1 angegeben, gemessen. Dabei wurde festgestellt, daß die Nukleotide 2, 7, 8, 9, 11, 14 und 15 ohne Aktivftätsverlust gegen modifzierte Nukleotide ausgetauscht werden konnten (Fig. 6). Es ergab sich sogar ein Aktivitätsgewinn von mindestens 20%.

Weiterhin wurden die 6 Nukleotide bei DH5 zusammen gegen die 2'-O-Methyl-Ribose-modifizierten Nukleotide ausgestauscht (Konstrukt CM6). Dabei blieb die Aktivität gegenüber dem unmodifizierten Konstrukt im wesentlichen erhalten (Fig. 4; vgl. CM6 mit DH5-9).

Beim DNA-Enzym gegen den VR1 konnten die obigen Nukleotide der Kemsequenz zusammen modifiziert werden, wobei die Aktivität gegenüber dem unmodifizierten Konstrukt nicht nur erhalten blieb, sondern sogar eine gesteigerte Aktivität beobachtet wurde:
- DV15 (unmod.):: 0,5 ± 0,1 nM/min
- DV15-CM6:: 0,8 ±.0,2 nM/min

### Beispiel 4

### Einfluß der Kombination von Kernsequenz- und Substraterkennungsarm-Modifikationen auf die Aktivität von DNA-Enzymen

Des weiteren wurde die Anfangsreaktimmsgmüwindigkeit unter MTO-Bedingungen beim DNA-Enzym gegen humanen Rhinovirus 14 mit 7 Nukleotide langen Substraterkennungsarmen untersucht, bei dem die Nukleotide 2, 7, 8, 11, 14 und 15 der Kernsequenz sowie jeweils vom Ende der Substraterkennungsarme 5 Nukleotide gegen entsprechende 2'-O-Methyl-Ribonukleotide ausgetauscht wurden.

Dabei ergab sich eine fast 10fach erhöhte Aktivität des modifizierten, d.h. vollständig stabilisierten, gegenüber dem unmodifizierten Konstrukt:
- DH5-9 (unmod.):: 0,21 ± 003 nM/min
- DH5-9 (vollst. stab.):: 2,0 ± 0,1 nM/min

### Beispiel 5

### Kinetische Analyse mit langer Ziel-RNA ("long target RNA")

Die kinetischen Experimente mit "long target RNA" wurden in 50 mM Tris-HCl, 10 mM MgCl₂ und 1 U/µl RNAsin durchgeführt, um unspezifische RNA-Degradation zu vermeiden. Die DNAzyme wurden für 2 Minuten bei 65°C denaturiert und dann auf 37°C gekühlt. Die Reaktionen wurden durch Hinzugabe von DNAzym zu 100 nM "long target" Lösung gestartet. Die Enzymkonzentration für einzelne und multiple "turn over" Experimente waren 1µM und 10 nM. Aliquots wurden nach definierten Intervallen während der ersten 10% der Reaktion im Falle multipler "turn over" Bedingungen und während einer verlängerten Periode für Einzel-"turn over"-Untersuchungen abgenommen. Die Reaktion wurde durch Hinzugabe von 83 mM EDTA und Eiskühlung gestoppt. Die Spaltungsreaktionen wurden durch Agarosegelelektrophorese und Ethidiumbromid-Markierung analysiert. Die Bandenintensitäten wurden mit Hilfe der Quantity One Software (Bio-Rad, München, Deutschland) quantifiziert. Die Daten wurden weiter durch "fitting" analysiert (entweder linear zum Erhalt einer initialen Geschwindigkeit Vᵢₙᵢₜ für Substratüberschußexperimente oder mit einer Einzelexponentialfunktion zum Erhalt der beobachteten Spaltungsgeschwindigkeit für Enzymüberschußexperimente und Verwendung von Origin (Microcal Software, Northampton, MA). Die Werte sind Mittelwerte ± Standardabweichung von mindestens 3 unabhängigen Experimenten.

### Stabilitätsassay

Resistenz gegenüber nukleolytischem Abbau von verschiedenen DNAzymen im Zellkutturmedium wurde bewertet DNAzym (1 µM) wurde in DMEM (Cytogen, Sinn, Deutschland), enthaltend 10% FCS (PAA Laboratorien, Linz, Österreich) bei 37°C inkubiert. Die Proben wurden zu definierten Zeitpunkten zwischen 0 und 72 Stunden abgenommen und die andauernden Reaktionen wurden durch die Hinzugabe von einer gleichen Menge von 9 M Harnstoff in TBE und nachfolgendem Einfrieren in flüssigem Stickstoff unterbrochen. Die Oligonukleotide wurden Phenol-extrahiert und bei -20°C über Nacht durch Hinzugabe von Natriumacetat pH 5,2 präzipitiert, so daß eine Endkonzentration von 0,3 M und Zugabe von 2,5 vol Ethanol erfolgte. Das Präzipitat wurde mit 70% Ethanol gewaschen und in einer geeigneten Menge Wasser resuspendiert. Nach Denaturierung für 5 Minuten bei 85°C wurden Abbauprodukte auf 20%igem denaturierendem Polyacrylamidgel separiert. Weitere Analyse wurde unter Verwendung des Quantity One Programms (Bio-Rad, München, Deutschland) durchgeführt. Die Halbwertszeiten von DNAzym wurden durch "fitting" der Menge des Volllängenoligonukleotids bei verschiedenen Zeitpunkten zu einer Exponentionalfunktion erster Ordnung unter Verwendung von Origin (Microcal Software, Nortampton, MA) erhalten.

Um die Stabilität von DNAzym gegenüber endonukleolytischem Abbau zu messen, wurden 2 µM Oligonukleotide mit 0,4 U S1 Endonuklease (Promega, Madison, WI) pro 100 pmol DNAzym im Puffer des Herstellers (50 mM Natriumacetat, pH 4,5, 280 mM NaCl, 4,5 mM ZnSO₄) inkubiert. Aliquots wurden nach definierten Zeiten in Intervallen zwischen 0 und 180 Minuten abgenommen. Die Reaktionen wurden durch Erhitzung auf 98°C für 3 Minuten und nachfolgendes Einfrieren in flüssigem Stickstoff unterbrochen. Die Oligonukleotide wurden durch Ethanol präzipitiert und weiterbehandelt, wie oben für den Stabilitätsassay im Zellkulturmedium beschrieben. Die angegebenen Werte sind Durchschnittswerte Standardabweichung von mindestens 3 unabhängigen Experimenten.

**Tab.2**

| *DNAzym* | *Armlänge* | *Modifikation* | *T_{M} (°C)* | *k_{obs} (min⁻¹)* | *vᵢₙᵢₜ (nM*min⁻¹)* |
|---|---|---|---|---|---|
| DH5-9/0 | 9 | keine | 32 | 0.057±0.005 | 0.21±0.03 |
| DH5-7/0 | 7 | keine | n.d. (<25) | 0.033±0.002 | n.d. (<0.05) |
| DH5-iT | 9 | invertiertes T am 3' Ende | 33 | 0.052±0.005 | 0.19±0.01 |
| DH5-Thio | 9 | all-Phosphorothioat Bindungsarme | 27 | 0.009±0.001 | n.d.(<0.05) |
| LH5-9/4 | 9 | 4 LNA Endblöcke | 63 | 0.24±0.02 | 0.08±0.02 |
| LH5-7/3 | 7 | 3 LNA Endblöcke | 48 | 0.45±0.01 | 1.2±0.1 |
| LH5-7/4 | 7 | 4 LNA Endblöcke | 61 | 0.44±0.05 | 0.45±0.09 |
| DH5-OMe9/4 | 9 | 4 OMe Endblöcke | 47 | 0.11±0.01 | 1.2±0.1 |
| DH5-OMe8/4 | 8 | 4 OMe Endblöcke | 44 | 0.29±0.01 | 2.8±0.5 |
| DH5-OMe7/3 | 7 | 3 OMe Endblöcke | 32 | 0.12±0.03 | 3.8±0.3 |
| DH5-OMe7/4 | 7 | 4 OMe Endblöcke | 37 | 0.31±0.01 | 4.7±0.4 |
| DH5-0Me7/5 | 7 | 5 OMe Endblöcke | 39 | 0.5±0.1 | 4.7±0.9 |
| DH5-OMe7/6 | 7 | 6 OMe Endblöcke | 44 | 0.23±0.06 | 1.9±0.5 |
| DH5-OMe7/7 | 7 | 7 OMe Endblöcke | 47 | 0.027±0.006 | 0.21±0.02 |
| DH5-OMe6/5 | 6 | 5 OMe Endblöcke | 26 | 0.17±0.01 | 1.1±0.2 |
| DH5-CM6 | 9 | 6 OMe im katalytischen Zentrum | 31 | 0.06±0.01 | 0.09±0.01 |
| DH5 E | 7 | 5 OMe Endblöcke; 6 OMe im katalytischen Zentrum | 39 | 0.57±0.07 | 2.0±0.2 |
| DV15 9/0 | 9 | keine | 37 | 0.9±0.1 | 0.5±0.1 |
| DV15-OMe 7/5 | 7 | 5 OMe Endblöcke | 40 | 0.83±0.07 | 1.7±0.2 |
| DV15-CM6 | 9 | 6 OMe im katalytischen Zentrum | 36 | 0.43±0.03 | 0.8±0.2 |
| DV15 E | 7 | 5 OMe Endblöcke; 6 OMe im katalytischen Zentrum | 37 | 0.05±0.01 | n.d.(<0.05) |
| DV15 E4 | 7 | 4 OMe Endblöcke, 6 OMe im katalytischen Zentrum | 36 | 0.31±0.08 | 1.3±0.2 |

In Tabelle 2 sind die untersuchten DNAzyme mit ihrer jeweiligen Armlänge und den entsprechend vorgenommen-Modifikationen-dargestellt. Weiterhin enthält Tabelle 2 den Schmelzpunkt (Tₘ (°C)) und die beobachteten Spaltungsgeschwindigkeiten (min⁻¹) (k_{obs}) in Einzel-"turn over"-Experimenten und die initialen Geschwindigkeiten (vᵢₙᵢₜ) in multiplen "turn oder" Experimenten (jeweils mit "long target" RNA). In den Bezeichnungen der DNAzyme ist vor dem Schrägstrich jeweils die Anzahl der Nukleotide in jedem Bindungsarm genannt. Die Angabe nach dem Schrägstrich bezieht sich auf die Anzahl der modifizierten Nukleotide in jedem Bindungsarm. Die Abkürzung OMe steht für die 2'-O-Methyl-Modifikation. Die Abkürzung iT steht für 3'-invertiertes Thymidin. Thio bedeutet, daß die Bindungsarme alle Phosphorthioate enthalten. L steht für LNA-Modifikation. Tₘ gibt die Schmelztemperaturen der Zielmolekül/Enzym-Duplexe an.

**Tab. 3**

| | *vᵢₙᵢₜ* | *t*_{*1*/}*₂Medium* | *t*_{*1*/}*₂ S1-Nucl.* | *Performance index* |
|---|---|---|---|---|
| DH5-9/0 | 1 | 1 | 1 | 1 |
| DH5-Thio | <0.23 | 11.5 | 1.6 | <4 |
| DH5-iT | 0.9 | 11.5 | 1 | 10 |
| DH5-OMe7/5 | 22.4 | 3.5 | 1 | 78 |
| LH5-7/3 | 5.7 | 9.5 | 1.5 | 81 |
| DH5 E | 9.7 | 12.5 | 2.1 | 255 |

Tabelle 3 enthält eine Zusammenfassung der Ergebnisse für verschiedene modifizierte DNAzyme im Vergleich. Hierbei ist die Initialgeschwindigkeit unter den verschiedenen turn over Bedingungen, nämlich im Zellkulturmedium und die Stabilität gegenüber Endonuklease S1 angegeben. Alle Werte sind normalisiert gegenüber unmodifizertem DNAzym. Ein Index des Gesamtergebnisses der modifizierten DNAzyme aus drei Werten ist in der letzten Spalte angegeben. In Hinblick auf die verwendeten Abkürzungen wird auf Tabelle 2 bzw. die zugehörigen Erläuterungen verwiesen.

## Patentansprüche

1. DNA-Enzym vom Typ 10-23, umfassend von 5' nach 3' einen ersten Substraterkennungsarm (Abschnitt I), eine katalytische Kernsequenz (Abschnitt II) und einen zweiten Substraterkennungsarm (Abschnitt III), wobei in Abschnitt II lediglich eines öder mehrere der Nukleotide 2, 7, 8, 11, 14 und 15 chemisch modifiziert ist/sind und wobei das/die modifizierte(n) Nukleotid(e) aus der Gruppe, bestehend aus Phosphorthioat-Nukleotiden, invertiertem Thymidin, 2'-O-Methyl-Ribonukleotiden und LNA-Ribonukleotiden, ausgewählt ist/sind.

2. DNA-Enzym nach Anspruch 1, wobei alle der Nukleotide 2, 7, 8, 11, 14 und 15 des Abschnitts II modifiziert sind.

3. DNA-Enzym nach Anspruch 1 oder 2, wobei eines oder mehrere der Nukleotide des Abschnitts I und/oder des Abschnitts III modifiziert ist/sind.

4. DNA-Enzym nach Anspruch 3, wobei 3 bis 5 Nukleotide des Abschnitts I und/oder des Abschnitts III modifiziert sind.

5. DNA-Enzym nach Anspruch 4, wobei sich die modifizierten Nukleotide am 5'-Ende des Abschnitts I und/oder am 3'-Ende des Abschnitts III befinden.

6. DNA-Enzym nach Anspruch 4 oder 5, wobei die modifizierten Nukleotide des Abschnitts I und/oder des Abschnitts III 2'-O-Methyl-Ribonukleotide oder LNA-Ribonukleotide sind.

7. DNA-Enzym nach einem der Ansprüche 1 bis 6, wobei der Abschnitt I und/oder der Abschnitt III nicht mehr als 8 Nukleotide umfasst.

8. DNA-Enzym nach Anspruch 7, wobei der Abschnitt I und/oder der Abschnitt III 7 Nukleotide umfasst.

9. DNA-Enzym nach Anspruch 3 wobei alle Nukleotide des Abschnitts I und/oder des Abschnitts III Phosphorthioat-Nukleotide oder 2'-O-Methyl-Ribonukleotide sind.

10. DNA-Enzym nach einem der Ansprüche 3 bis 9, wobei die Schmelztemperatur der zwischen den Abschnitten I und III und dem Zielmolekül gebildeten Doppelstränge etwa 33 bis etwa 42°C beträgt.

11. DNA-Enzym nach einem der Ansprüche 1 bis 10, wobei der Abschnitt II die folgende Konsensussequenz von 5' nach 3' aufweist:
GGMTMGH (N) DNNNMGD
mit
M = A oder C;
H= A, C, oder T;
D=G, A oder T; und
N = irgendeine Base.

12. DNA-Enzym nach einem der Ansprüche 1 bis 11, das gegen die mRNA des Vanilloid-Rezeptors 1 (VR1) gerichtet ist.

13. DNA-Enzym nach Anspruch 12, wobei die Abschnitte I und III von 5' nach 3' die folgenden Sequenzen oder eine in einem Nukleotid davon abweichende Sequenz aufweisen, mit der Maßgabe, dass sich das von den angegebenen Sequenzen abweichende Nukleotid nicht an einer der letzten drei Positionen des Abschnitts I und nicht an einer der ersten 3 Positionen des Abschnitts III befindet :
| Abschnitt I | Abschnitt III |
|---|---|
| GTCATGA | GGTTAGG |
| TGTCATGA | GGTTAGGG |
| ATGTCATGA | GGTTAGGGG |
| GTCGTGG | GGTTAGG |
| TGTCGTGG | GATTAGG |
| ATGTCGTGG | GATTAGG |
| TTGTTGA | GGTCTCA |
| CTTGTTGA | GGTCTCAC |
| TCTTGTTGA | GGTCTCACC |
| TTGTTGA | AGTCTCA |
| CTTGTTGA | AGTCTCAN |
| TCTTGTTGA | AGTCTCANN |
| GGCCTGA | CTCAGGG |
| CGGCCTGA | CTCAGGGA |
| TCGGCCTGA | CTCAGGGAG |
| TGCTTGA | CGCAGGG |
| CTGCTTGA | CGCAGGGN |
| TCTGCTTGA | CGCAGGGNN |
| GTGTGGA | TCCATAG |
| GGTGTGGA | TCCATAGG |
| TGGTGTGGA | TCCATAGGC |
| ACGTGGA | TCAGACG |
| GACGTGGA | TCAGACGN |
| CGACGTGGA | TCAGACGNN |
| GTGGGGA | TCAGACT |
| GGTGGGGA | TCAGACTC |
| GGGTGGGGA | TCAGACTCC |
| GTGGGTC | GCAGCAG |
| AGTGGGTC | GCAGCAG |
| GAGTGGGTC | GCAGCAG |
| CGCTTGA | AAATCTG |
| CGCTTGA | AAATCTGT |
| TGCGCTTGA | AAATCTGTC |
| CGCTTGA | GAATCTG |
| GCGCTTGA | GAATCTGN |
| TGCGCTTGA | GAATCTGNN |
| CTCCAGA | ATGTGGA |
| GCTCCAGA | ATGTGGAA |
| AGCTCCAGA | ATGTGGAAT |
| GCTCCAGG | AGGTGGA |
| GCTCCAGG | AGGTGGA |
| AGCTCCAGG | AGGTGGA |
| GGGTACGA | TCCTGGT |
| GGGTACGA | TCCTGGTA |
| CGGGTACGA | TCCTGGTAG |
| GGTGCGG | TCTTGGC |
| GGGTGCGG | TCTTGGC |
| CGGGTGCGG | TCTTGGC |
mit N = irgendeiner Base

14. Wirtszelle, enthaltend mindestens ein DNA-Enzym nach einem der Ansprüche **1** bis **13,** wobei die Wirtszelle keine humane Keimzelle und keine humane embryonale Stammzelle ist.

15. Wirtszelle nach Anspruch **14,** die eine Säugetierzelle ist.

16. Wirtszelle nach Anspruch **15,** die eine humane Zelle ist.

17. *Ex vivo* Verfahren zur Herunterregulation der Expression eines Gens, umfassend das Einbringen, mindestens eines DNA-Enzyms nach einem der Ansprüche **1** bis **15** in eine das Gen exprimierende Zelle.

18. *Ex vivo* Verfahren nach Anspruch **17,** wobei das Gen das VR1-Gen ist und mindestens ein DNA-Enzym nach Anspruch **12** oder **13** in die Zelle eingebracht wird.

19. Arzneimittel, enthaltend mindestens ein DNA-Enzym nach einem der Ansprüche 1 bis **13** und/oder eine Wirtszelle nach einem der Ansprüche 14 bis 16, gegebenenfalls in Verbindung mit einem oder mehreren pharmazeutisch verträglichen Trägern und/oder Vehikeln.

20. Verwendung des DNA-Enzyms nach Anspruch **12** oder **13** oder der Wirtszelle nach einem der Ansprüche **14** bis **16** zur Herstellung eines Arzneimittels zur Prävention und/oder Behandlung von Schmerz, insbesondere chronischem Schmerz, taktiler Allodynie, thermisch ausgelöstern Schmerz und/oder Entzündungsschmerz.

21. Verwendung des DNA-Enzyms nach Anspruch **12** oder **13** oder der Wirtszelle nach einem der Ansprüche **14** bis **16** zur Herstellung eines Arzneimittels zur Prävention und/oder Behandlung von neurogenen Blasensymptomen, Harninkontinenz, VR1-assoziierten Sensibilitätsstörungen, VR1-assoziierten Entzündungen und VR1-assoziierten Tumoren.

## Claims

1. A DNA enzyme of type 10-23, comprising, in 5' to 3' direction, a first substrate recognition arm (segment I), a catalytic core sequence (segment II) and a second substrate recognition arm (segment III), wherein in segment II, only one or more of nucleotides 2, 7, 8, 11, 14 and 15 is/are chemically modified and wherein the modified nucleotide(s) is/are selected from the group consisting of phosphorothioate nucleotides, inverted thymidine, 2'-O-methyl ribonucleotides and LNA ribonucleotides.

2. The DNA enzyme according to claim 1, wherein all of nucleotides 2, 7, 8, 11, 14 and 15 of segment II are modified.

3. The DNA enzyme according to claim 1 or 2, wherein one or more of the nucleotides of segment I and/or of segment III is/are modified.

4. The DNA enzyme according to claim 3, wherein 3 to 5 nucleotides of segment I and/or of segment III are modified.

5. The DNA enzyme according to claim 4, wherein the modified nucleotides are localized at the 5' end of segment I and/or at the 3' end of segment III.

6. The DNA enzyme according to claim 4 or 5, wherein the modified nucleotides of segment I and/or of segment III are 2'-O-methyl ribonucleotides or LNA ribonucleotides.

7. The DNA enzyme according to one of claims 1 to 6, wherein segment I and/or segment III comprise(s) not more than 8 nucleotides.

8. The DNA enzyme according to claim 7, wherein segment I and/or segment III comprise(s) 7 nucleotides.

9. The DNA enzyme according to claim 3, wherein all nucleotides of segment I and/or of segment III are phosphorothioate nucleotides or 2'-O-methyl ribonucleotides.

10. The DNA enzyme according to one of claims 3 to 9, wherein the melting temperature of the double strands formed between segments I and III and the target molecule is about 33 to about 42°C.

11. The DNA enzyme according to one of claims 1 to 10,
wherein segment II has, in 5- to 3- direction, the following consensus sequence:
GGMTMGH (N) DNNNMGD
wherein
M = A or C;
H = A, C, or T;
D = G, A or T; and
N = any base.

12. The DNA enzyme according to one of claims 1 to 11 which is directed against the mRNA of vanilloid receptor 1 (VR1).

13. The DNA enzyme according to claim 12, wherein segments I and III have, in 5' to 3' direction, the following sequences or a sequence differing in one nucleotide, with the proviso that the nucleotide differing from the indicated sequences is not located in one of the three last positions of segment I and is not located at one of the first three positions of segment III:
| Segment I | Segment III |
|---|---|
| GTCATGA | GGTTAGG |
| TGTCATGA | GGTTAGGG |
| ATGTCATGA | GGTTAGGGG |
| GTCGTGG | GATTAGG |
| | |
| TGTCGTGG | GATTAGG |
| ATGTCGTGG | GATTAGG |
| TTGTTGA | GGTCTCA |
| CTTGTTGA | GGTCTCAC |
| TCTTGTTGA | GGTCTCACC |
| TTGTTGA | AGTCTCA |
| CTTGTTGA | AGTCTCAN |
| TCTTGTTGA | AGTCTCANN |
| GGCCTGA | CTCAGGG |
| CGGCCTGA | CTCAGGGA |
| TCGGCCTGA | CTCAGGGAG |
| TGCTTGA | CGCAGGG |
| CTGCTTGA | CGCAGGGN |
| TCTGCTTGA | CGCAGGGNN |
| GTGTGGA | TCCATAG |
| GGTGTGGA | TCCATAGG |
| TGGTGTGGA | TCCATAGGC |
| ACGTGGA | TCAGACG |
| GACGTGGA | TCAGACGN |
| CGACGTGGA | TCAGACGNN |
| GTGGGGA | TCAGACT |
| GGTGGGGA | TCAGACTC |
| GGGTGGGGA | TCAGACTCC |
| GTGGGTC | GCAGCAG |
| AGTGGGTC | GCAGCAG |
| GAGTGGGTC | GCAGCAG |
| CGCTTGA | AAATCTG |
| GCGCTTGA | AAATCTGT |
| TGCGCTTGA | AAATCTGTC |
| CGCTTGA | GAATCTG |
| GCGCTTGA | GAATCTGN |
| TGCGCTTGA | GAATCTGNN |
| CTCCAGA | ATGTGGA |
| GCTCCAGA | ATGTGGAA |
| AGCTCCAGA | ATGTGGAAT |
| CTCCAGG | AGGTGGA |
| GCTCCAGG | AGGTGGA |
| AGCTCCAGG | AGGTGGA |
| GGTACGA | TCCTGGT |
| GGGTACGA | TCCTGGTA |
| CGGGTACGA | TCCTGGTAG |
| GGTGCGG | TCTTGGC |
| GGGTGCGG | TCTTGGC |
| CGGGTGCGG | TCTTGGC |
wherein N = any base.

14. A host cell comprising at least one DNA enzyme according to one of claims 1 to 13 wherein the host cell is no human germ cell and no human embryonal stem cell.

15. The host cell according to claim 14, being a mammalian cell.

16. The host cell according to claim 15, being a human cell.

17. An *ex vivo* method for down regulation of the expression of a gene, comprising introducing at least one DNA enzyme according to one of claims 1 to 15 in a cell expressing said gene.

18. The *ex vivo* method according to claim 17, wherein the gene is the VR1 gene and wherein at least one DNA enzyme according to claim 12 or 13 is introduced into said cell.

19. Medicament comprising at least one DNA enzyme according to one of claims 1 to 13 and/or a host cell according to one of claims 14 to 16, optionally in combination with one or more pharmaceutically acceptable carrier(s) and/or vehicle(s).

20. Use of the DNA enzyme according to claim 12 or 13 or of the host cell according to one of claims 14 to 16 in the manufacture of a medicament for the prevention and/or treatment of pain, in particular chronic pain, tactile alodynia, thermically induced pain and/or inflammatory pain.

21. Use of the DNA enzyme according to claim 12 or 13 or of the host cell according to one of claims 14 to 16 in the manufacture of a medicament for the prevention and/or treatment of symptoms of neurogenic bladder, urinary incontinence, VR1 associated sensitivity disorders, VR1 associated inflammations and VR1 associated tumors.

## Revendications

1. Enzyme à ADN du type 10-23 comprenant de 5' en 3' un premier bras de reconnaissance de substrat (segment I), une séquence centrale catalytique (segment II) et un second bras de reconnaissance de substrat (segment III), dans le segment II seulement l'un ou plusieurs des nucléotides 2, 7, 8, 11, 14 et 15 étant modifié(s) chimiquement et le nucléotide modifié/les nucléotides modifiés étant choisi(s) parmi le groupe consistant en les nucléotides modifiés au phosphorothioate, la thymidine invertie, les 2'-O-méthyl-ribonucléotides et les ribonucléotides de LNA.

2. Enzyme à ADN selon la revendication 1, dans laquelle tous les nucléotides 2, 7, 8, 11, 14 et 15 du segment II sont modifiés.

3. Enzyme à ADN selon la revendication 1 ou 2, dans laquelle l'un ou plusieurs des nucléotides du segment I et/ou du segment III est modifié/sont modifiés.

4. Enzyme à ADN selon la revendication 3, dans lequel 3 à 5 nucléotides du segment I et/ou du segment III sont modifiés.

5. Enzyme à ADN selon la revendication 4, dans laquelle les nucléotides modifiés sont présents à l'extrémité 5' du segment I et/ou à l'extrémité 3' du segment III.

6. Enzyme à ADN selon la revendication 4 ou 5, dans laquelle les nucléotides modifiés du segment I et/ou du segment III sont des 2'-O-méthyl-ribonucléotides ou des ribonucléotides de LNA.

7. Enzyme à ADN selon l'une quelconque des revendications 1 à 6, dans laquelle le segment I et/ou le segment III comporte/comportent au maximum 8 nucléotides.

8. Enzyme à ADN selon la revendication 7, dans laquelle le segment I et/ou le segment III comporte/comportent sept nucléotides.

9. Enzyme à ADN selon la revendication 3, dans laquelle tous les nucléotides du segment I et/ou du segment III sont des nucléotides modifiés au phosphorothioate ou des 2'-O-méthyl-ribonucléotides.

10. Enzyme à ADN selon l'une quelconque des revendications 3 à 9, dans laquelle la température de fusion des double bras formés entre les segments I et III et la molécule de cible est environ 33 à environ 42°C.

11. Enzyme à ADN selon l'une quelconque des revendications 1 à 10, dans laquelle le segment II a la séquence consensus suivante de 5' en 3':
GGMTMGH (N) DNNNMGD
où
M = A ou C;
H = A, C ou T;
D = G, A ou T; et
N = une base quelconque.

12. Enzyme à ADN selon l'une quelconque des revendications 1 à 11, dirigé contre le ARN messager du récepteur Vanilloid 1 (VR1).

13. Enzyme à ADN selon la revendication 12, dans laquelle le segment I et III comporte de 5' en 3' les séquences suivantes
ou une séquence différente d'un nucléotide comparé à ceux-ci, sous réserve que le nucléotide différent des séquences mentionnées ne soit pas à l'une des trois dernières positions du segment I et ne soit pas à l'une des trois premières positions du segment III.
| Segment I | Segment III |
|---|---|
| GTCATGA | GGTTAGG |
| TGTCATGA | GGTTAGGG |
| ATGTCATGA | GGTTAGGGG |
| TGTCGTGG | GATTAGG |
| TGTCGTGG | GATTAGG |
| ATGTCGTGG | GATTAGG |
| TTGTTGA | GGTCTCA |
| CTTGTTGA | GGTCTCAC |
| TCTTGTTGA | GGTCTCACC |
| TTGTTGA | AGTCTCA |
| CTTGTTGA | AGTCTCAN |
| TCTTGTTGA | AGTCTCANN |
| GGCCTGA | CTCAGGG |
| CGGCCTGA | CTCAGGGA |
| CGGCCTGA | CTCAGGGAG |
| TGCTTGA | CGCAGGG |
| CTGCTTGA | CGCAGGGN |
| TCTGCTTGA | CGCAGGGNN |
| GTGTGGA | TCCATAG |
| GGTGTGGA | TCCATAGG |
| TGGTGTGGA | TCCATAGGC |
| ACGTGGA | TCAGACG |
| GACGTGGA | TCAGACGN |
| CGACGTGGA | TCAGACGNN |
| GTGGGGA | TCAGACT |
| GGTGGGGA | TCAGACTC |
| GGGTGGGGA | TCAGACTCC |
| GTGGGTC | GCAGCAG |
| AGTGGGTC | GCAGCAG |
| GAGTGGGTC | GCAGCAG |
| CGCTTGA | AAATCTG |
| GCGCTTGA | AAATCTG |
| TGCGCTTGA | AAATCTGTC |
| CGCTTGA | GAATCTG |
| GCGCTTGA | GAATCTGN |
| TGCGCTTGA | GAATCTGNN |
| CTCCAGA | ATGTGGA |
| GCTCCAGA | ATGTGGAA |
| AGCTCCAGA | ATGTGGAAT |
| GCTCCAGG | AGGTGGA |
| GCTCCAGA | AGGTGGA |
| AGCTCCAGG | AGGTGGA |
| GGTACGA | TCCTGGT |
| CGGGTACGA | TCCTGGTA |
| CGGGTACGA | TCCTGGTAG |
| GGTGCGG | TCTTGGC |
| GGGTGCGG | TCTTGGC |
| GGGTGCGG | TCTTGGC |
où N = une base quelconque.

14. Cellule hôte contenant au moins une enzyme à ADN selon l'une quelconque des revendications 1 à 13, la cellule hôte n'étant pas une cellule gérminative d'origine humaine et n'étant pas une cellule souche embryonnaire d'origine humaine.

15. Cellule hôte selon la revendication 14, qui est une cellule de mammifière.

16. Cellule hôte selon la revendication 15, qui est une cellule humaine.

17. Procédé ex-vivo pour la régulation descendante de l'expression d'un gène comprenant l'introduction d'au moins une enzyme à ADN selon l'une quelconque des revendications 1 à 15 dans une cellule exprimant ledit gène.

18. Procédé ex-vivo selon la revendication 17, dans lequel le gène est le gène VR1 et au moins une enzyme à ADN selon la revendication 12 ou 13 est introduite dans la cellule.

19. Médicament, contenant au moins une enzyme à ADN selon l'une quelconque des revendications 1 à 13 et/ou une cellule hôte selon l'une quelconque des revendications 14 à 16, éventuellement en combinaison avec un ou plusieurs supports et/ou véhicules pharmaceutiquement acceptables.

20. Utilisation de l'enzyme à ADN selon la revendication 12
ou 13 ou de la cellule hôte selon l'une quelconque des revendications 14 à 16 pour la fabrication d'un médicament destiné à la prévention et/ou traitement de la douleur, en particulier de la douleur chronique, de l'allodynie tactile, de la douleur causée thermiquement et/ou de la douleur d'inflammation.

21. Utilisation de l'enzyme à ADN selon la revendication 12
ou 13 ou de la cellule hôte selon l'une quelconque des revendications 14 à 16 pour la fabrication d'un médicament destiné à la prévention et/ou au traitement des symptômes de la vessie neurogène, de l'incontinence urinaire, des troubles de sensibilité associés à VR1, des inflammations associées à VR1 et des tumeurs associées à VR1.
